# EUROPEAN PATENT SPECIFICATION

(11) **EP 1 104 272 B1**
(45) Date of publication and mention of the grant of the patent: **09.10.2002**
(21) Application number: 98939935.7
(22) Date of filing: 14.08.1998
(51) Int. Cl.: A61F 7/10

(54) **Apparatus for cooling the brain, brain stem and associated neurologic tissues**
Vorrichtung zur Kühlung des Gehirns, des Stammhirns und der assozierten neurologischen Gewebe
Dispositif de refroidissement du cerveau, du tronc cérébral et de tissus nuerologiques associés

(43) Date of publication of application: 06.06.2001
(73) Proprietor: Life Science Holdings, Inc., Des Plaines, IL 60018 (US)
(72) Inventor: KLATZ, Ronald, M., Chicago, IL 60614 (US); GOLDMAN, Robert, M., Chicago, IL 60614 (US)
(74) Representative: Guerre, Dominique
(86) International application number: US9816817
(87) International publication number: WO00009052

(56) References cited:
- EP-A- 0 449 299
- US-A- 4 920 963
- US-A- 5 261 399
- US-A- 5 755 756

## Description

### BACKGROUND OF THE INVENTION

### 1. Field of Invention

The invention relates generally to cooling of the brain, brain stem and associated tissues during trauma, or periods of decreased blood flow to reduce or prevent neurologic injuries thereto due to ischemia and anoxia. More particularly, the invention provides an apparatus and corresponding method for cooling of the brain, brain stem and associated neurologic tissues and maintaining them at a temperature sufficient to slow metabolic rates of the brain, brain stem and associated tissues a sufficient amount to reduce or prevent neurologic injuries thereto due to ischemia and anoxia. With the invention, the victim recovers with increased chances of survival and less chance of permanent brain damage.

### 2. Description of Related Art

When an ischemic or anoxic injury occurs, the brain is deprived of freshly oxygenated blood. For example, this situation typically occurs during cardiac arrest, respiratory arrest, stroke and other cerebrovascular trauma, suffocation, drowning, strangulation, electrocution, toxic poisoning (carbon monoxide, cyanide, etc.), metabolic insults or other similar trauma. Without a steady supply of freshly oxygenated blood, the brain ceases to function and after resuscitation, most patients will suffer some damage to the brain and associated neurologic tissues.

For example, among cardiac arrest victims overall less than 10% survive neurologically intact and without significant brain damage. The other approximately 90% either die or sustain some neurologic injury from ischemia (i.e., lack of blood flow to the brain), or anoxia (i.e., lack of oxygen to the brain). Such frequency of neurologic injury occurs because after a cardiac arrest, basic cardiopulmonary resuscitation and advanced life support techniques, such as CPR, closed heart cardiac chest massage, and electroshock treatments, typically require fifteen to twenty minutes to regain circulation from a failed heart. Reversible neurologic damage begins as early as four minutes and irreversible neurologic damage begins as early as six minutes after circulation stops. To combat this potential neurologic injury, initial resuscitation efforts need to be directed toward reviving the brain in addition to resuscitating the heart.

As indicated above, anoxic and ischemic brain injuries from cardiac arrest result in damage to the brain and associated neurologic tissues after about four minutes. In contrast, the heart can survive intact up to four hours after cardiac arrest. The short viability of brain tissue upon deprivation of oxygenated blood is a result of the requirement of high amounts of nutrients for tissue maintenance. Brain tissue uses almost all of the nutrients supplied by the circulating blood for maintenance and stores only a small amount of nutrients. Absent blood flow to the brain, the small amount of stored nutrients is rapidly exhausted. Once exhausted, brain oxygen content is rapidly depleted. This oxygen depletion is traumatic and causes a series of reactions in the oxygen starved brain tissue cells. These reactions are believed to produce free radical ions, primarily consisting of the superoxide radical O₂⁻. These free radicals complex with proteins in the brain and associated neurologic tissues, altering respiration, energy transfer and other vital cellular functions, and irreversibly damage these tissues.

Efforts should be directed toward resuscitating the brain to attempt to extend the period of time the brain can function without oxygen while the patient remains neurologically intact. The medical literature is replete with examples of humans surviving extended periods of time (greater than 5 minutes) without oxygen being delivered to the brain.

Hypothermic therapy is one method of keeping the brain alive absent oxygen. It involves cooling the brain to a temperature where its metabolic activity is decreased. When the brain's metabolic activity is decreased, it uses much less oxygen and stored nutrients are exhausted slowly, while production of irreversibly damaging O₂- free radicals is slowed and almost completely ceased. Thus, upon resuscitating the body from trauma, the patient emerges neurologically intact. For example, children revived after hours of submersion in very cold water have fully recovered with little if any neurologic damage.

Previous inventions, such as those described in U.S. Patents Nos. 5,149,321 to Klatz et al. ('321), 5,234,405 to Klatz et al. ('405), 5,261,399 to Klatz et al that discloses a device according to the first part of claim 1. ('399) and co-pending U.S Application No. 08/447,812 ('812), address the need to direct resuscitation efforts toward the brain, such that the victim can survive ischemic or anoxic trauma neurologically intact. Specifically, the '321 and '405 patents discuss devices and methods for resuscitating the brain such that its metabolism is slowed in order that the victim survive these metabolic insults neurologically intact. The '399 patent and the '812 application disclose a device and method for externally cooling the brain and associated tissues.

Russian Patents Nos. 1138152 ('152) and 904,695 ('695) disclose helmets for treatment of the head with cold or heat. However, the helmets of the '152 and '695 patents are not suited for field use, as they are large structures restricted to clinical facilities. Moreover, they must be used by a skilled surgical team and maintained by skilled technicians and are not disposable. Further, neither of these devices is configured to cool the neck and upper back.

Brain cooling devices, such as those disclosed in U.S. Patent No. 4,920,963 and Russian Patents Nos. 652,942 ('942), 454,907 ('907) and 446,015 ('015), are complex and require that the patient be placed into the device lying down. The '907 patent discloses using chilled water to effectuate cooling and the '942 and '015 patents are silent as to whether they have the capability of chilling the brain, brain stem and associated neurologic tissues a sufficient amount to reduce or prevent ischemic and anoxic injuries thereto. Further, none of these devices are disposable or suitable for field use due to their structure. Nor are any of these devices configured to cool the neck and upper back.

Various other patent documents disclose apparatus for cooling the head, for example, International Patent Publication No. WO 82/04184 ('184), International Patent Publication No. WO 89/09583 ('583), U.S. Patent No. 4,753,242 ('242), British Patent No. 2,130,489 ('489), German Patent No. 8607793 ('793), and U.S. Patents Nos. 3,909,655 ('655), 3,606,890 ('890), 4,691,762 ('762) and 4,138,743 ('743). These devices cool the head respectively to treat chemotherapy patients suffering from alopecia (hair loss); to treat post-operative plastic surgery patients; to treat patients suffering from migraines; or merely to provide a beauty treatment or for personal comfort. However, these devices are not capable of providing the cooling sufficient to slow metabolic rates of the brain, brain stem and associated tissues a sufficient amount to reduce or prevent neurologic injuries thereto due to ischemia and anoxia. Further, these devices are generally complicated, designed for clinical use, not disposable and are not configured to cool the neck and upper back.

### SUMMARY OF THE INVENTION

It is an object of the invention to provide an apparatus according to claim 1 and its dependent claims.

### BRIEF DESCRIPTION OF THE DRAWINGS

These and other aspects and advantages of the invention will become apparent from the following detailed description of preferred embodiments when taken in conjunction with the accompanying drawings, in which:
Fig. 1 is a front view of an embodiment of a cooling apparatus according to the invention;
Fig. 2 is a side view of the cooling apparatus of Fig. 1;
Fig. 3 is a top plan view of the cooling apparatus of Fig. 1;
Fig. 4 is a top plan view of the cooling apparatus of Fig. 1 employing an alternative coolant source;
Fig. 4A is a top plan view of the cooling apparatus of Fig. 1 employing another alternative coolant source;
Fig. 5 is a top plan view of the cooling apparatus of Fig. 1 employing another alternative coolant source;
Fig. 6 is a cross-sectional view of the cooling apparatus of Fig. 1 taken along line IV-IV of Fig. 3;
Fig. 7 is a front view of another embodiment of the cooling apparatus according to the invention;
Fig. 8 is a side view of the cooling apparatus of Fig. 7;
Fig. 9 shows the cooling apparatus of Fig. 1 with an eye flap;
Fig. 10 is a front view of still another embodiment of the cooling apparatus according to the invention;
Fig. 11 is a side view of the cooling apparatus of Fig. 10;
Fig. 12 is a cross-sectional view of the cooling apparatus of Fig. 10 taken along line XII-XII of Fig. 10;
Fig. 13 is a front view of yet another embodiment of the cooling apparatus according to the invention;
Fig. 14 is a side cross-sectional view of the cooling apparatus of Fig. 12; and
Fig. 15 is a cross-sectional view of the cooling apparatus of Fig. 13 taken along line XV-XV of Fig. 13.

### DETAILED DESCRIPTION OF PREFERRED EMBODIMENTS

While the invention will hereinafter be described in connection with preferred embodiments thereof, it will be understood that it is intended to limit the invention to those embodiments that may be included within scope of the invention as defined by the appended claims.

For a general understanding of the features of the invention, reference is made to the drawings. In the drawings, like reference numerals have been used throughout to designate like elements.

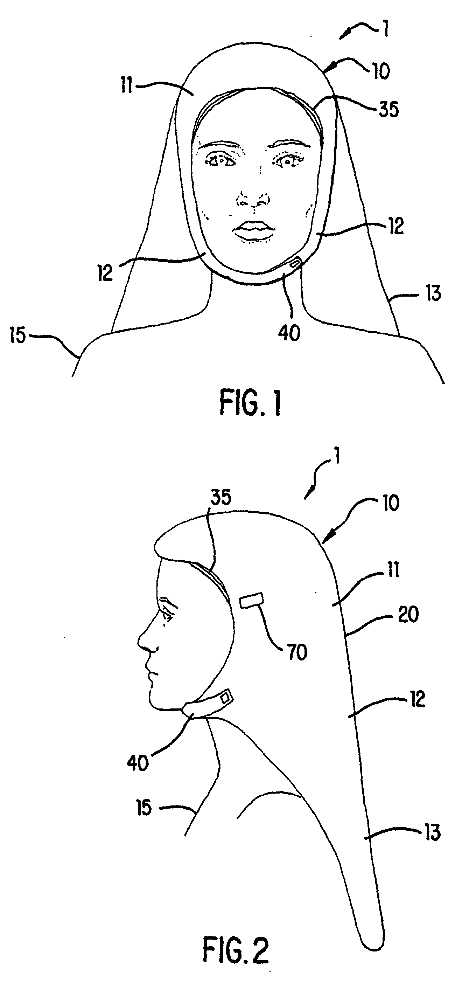

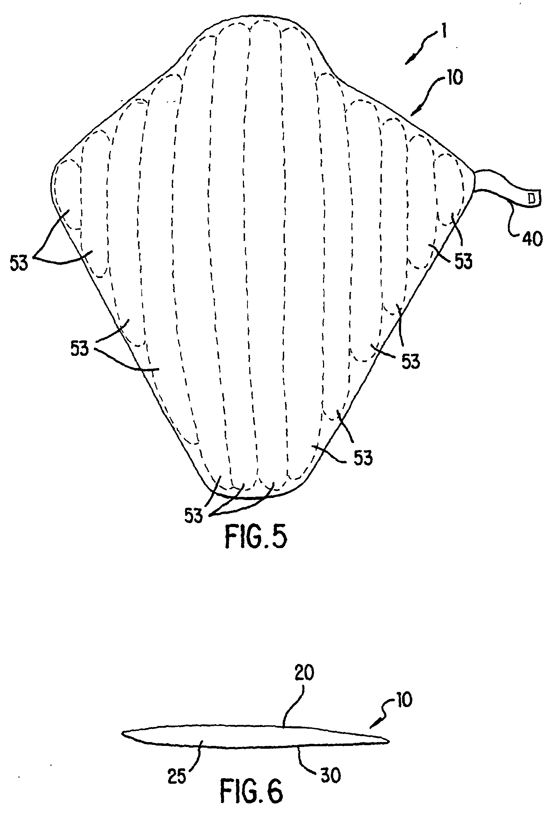

Fig. 1 is a front view of a preferred embodiment of the cooling apparatus according to the invention, while Fig. 2 is a side view of the cooling apparatus of Fig. 1. The cooling apparatus 1 shown in Fig. 1 is preferably formed of a scarf-shaped enveloping member 10. The enveloping member 10 is formed by outer 20 and inner 30 shell members, which are sealed together along their outer peripheral edges to form a cavity 25 (see Fig. 6). The enveloping member 10 preferably includes a head-enveloping portion 11, a neck enveloping portion 12 and an upper back-enveloping portion 13, preferably connected in series.

The outer shell member 20 and the inner shell member 30 are preferably made of a flexible, inexpensive material so that the enveloping member 10 may be used once and then discarded. The material may also include such materials as TEFLON®, TYVEC®, Gore-tex®, nylon, rubber or any non-porous flexible material.

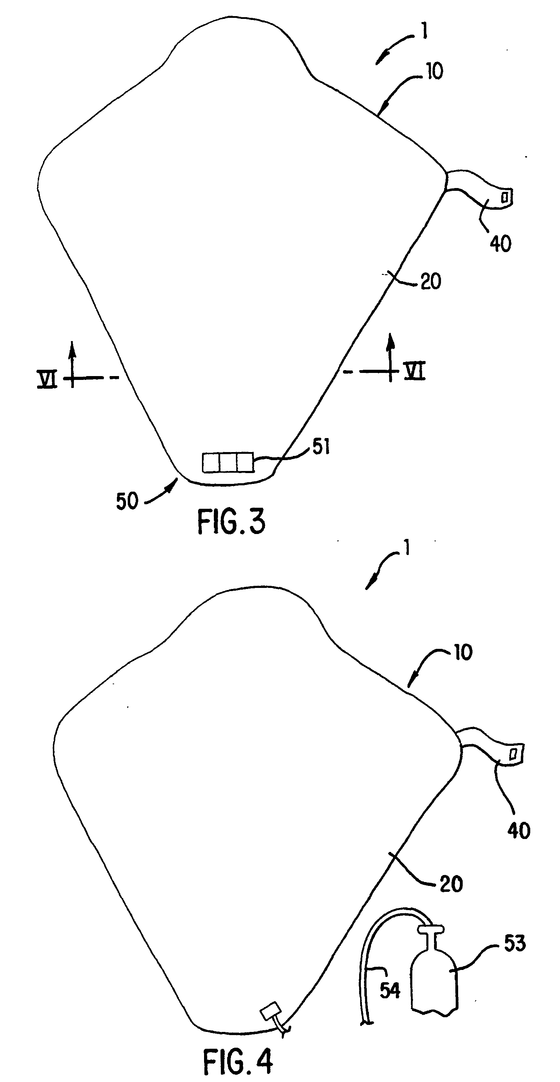

A coolant source 50 is configured to supply the. cavity 25 with coolant fluid chilled to a temperature sufficient to slow the metabolic rates of the brain, brain stem and associated tissues of a patient 15 a sufficient amount to reduce or prevent neurologic injuries thereto due to ischemia and anoxia. The coolant source 50 can be in the form of a charging mechanism 51 disposed on an outer surface of the outer shell member 20, as shown in Fig. 3. Such a charging mechanism 51 could contain compressed gas that would expand into the cavity 25 upon activation to provide the chilled coolant fluid.

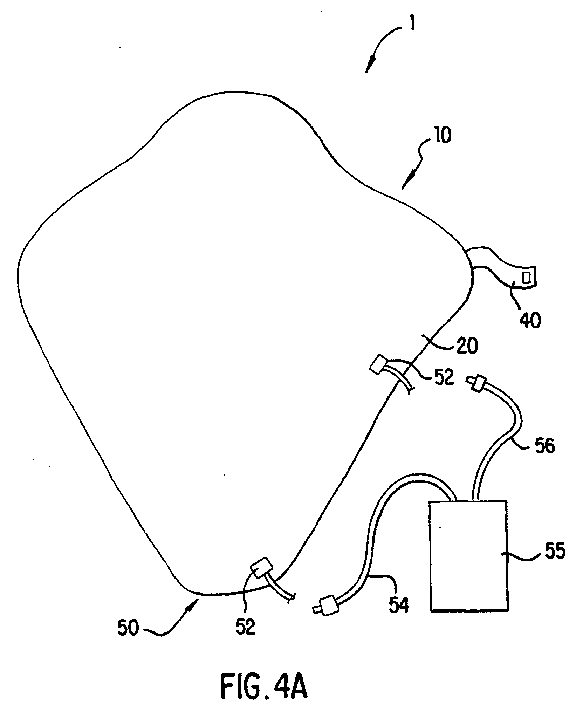

Alternatively, the coolant source 50 may comprise cold compressed liquids, such as, for example, carbon dioxide, freon or nitrogen, which upon decompression become cold gases, preferably stored in a portable container 53, such as, for example, a cartridge or tank (see Fig. 4). The gas preferably turns to dry ice upon expansion. The cavity 25 could also contain materials which chill upon activation when use is desired. For example, the cavity could be prefilled with a chemical, such as ammonium nitrate or an equivalent thereof, which reacts endothermically when activated by another chemical (e.g., water), chilling the device, or may contain materials activatable when exposed to a catalyst or enzyme activator. Alternatively, very cold liquids such as supercooled water, self freezing gel, packed liquid, ice water, or other such chemicals may be passed into the apparatus 10 through a tube 54 operatively connected to the nipple 52, or chilled by a refrigeration unit 55 which provides for two-way flow or circulation of the chilled fluid though the enveloping member 10 (see Fig. 4A). The refrigeration unit 55 could be computer controlled via a microprocessor in communication with a temperature sensor or sensors (not shown) which monitors the temperature in the enveloping member 10. The coolant source 50 may also be chemical packets 53 disposed within the cavity 25, which are activated when a membrane dividing the cavity into two or more chemical containing compartments is broken to allow the chemicals to mix (see Fig. 5), or which contain an ice-like material, such as blue ice, which is chilled in a refrigeration unit (not shown) until the apparatus is put to use. Additionally, the enveloping member 10 could include a quick charge valve acting as a coolant source to allow the device (not shown) to be rapidly filled with a coolant fluid, such as common crushed ice or snow.

In the case where the coolant source 50 is the charging mechanism, or the portable container 53, the outer shell member 20 may be provided with exhaust ports (not shown) to allow exit of the coolant fluid from the enveloping member 10. Further, the enveloping member 10 may be provided with means for collecting the exhausted coolant fluid for later disposal. Preferred coolant sources 29 are capable of generating temperatures as low as -24 degrees Celsius, and preferably temperatures approximately - 51 degrees Celsius or below.

Figs. 1-5 show a flexible closure device 40. The flexible closure device 40 should be elastic enough to allow for adjustment to various head sizes, yet resilient enough to maintain the enveloping member 10 on the head of the patient 15. The closure device 40 is preferably formed of quick release structure such as VELCRO®, or other suitable fastening materials. Mechanisms such as zippers, combinations of VELCRO® and zippers, or other equivalent fasteners can also be used to make this connection.

Further, the head enveloping portion 11 may be provided with a tightening mechanism 35, such as a draw string or elastic, for tightening the head enveloping portion 11 around the exposed face of the patient 15.

The inner layer of the inner shell member 30 may be coated with gel, such as any commercially available EKG electrode gel or ultrasound gel. The gel would be retained under paper, wax-based or TYVEK® type sheets, that peel off when use of the apparatus is desired.

As discussed above, a temperature sensor or sensors (not shown) and a microprocessor (not shown) may be used to allow control of the refrigeration unit 55, which would maintain the temperature within the enveloping member 10 by controlling a valve (not shown) to provide fresh chilled coolant fluid when needed. Further, the enveloping member 10 may be provided with a temperature display 70to allow one to assess the temperature at which the cooling apparatus 1 is performing.

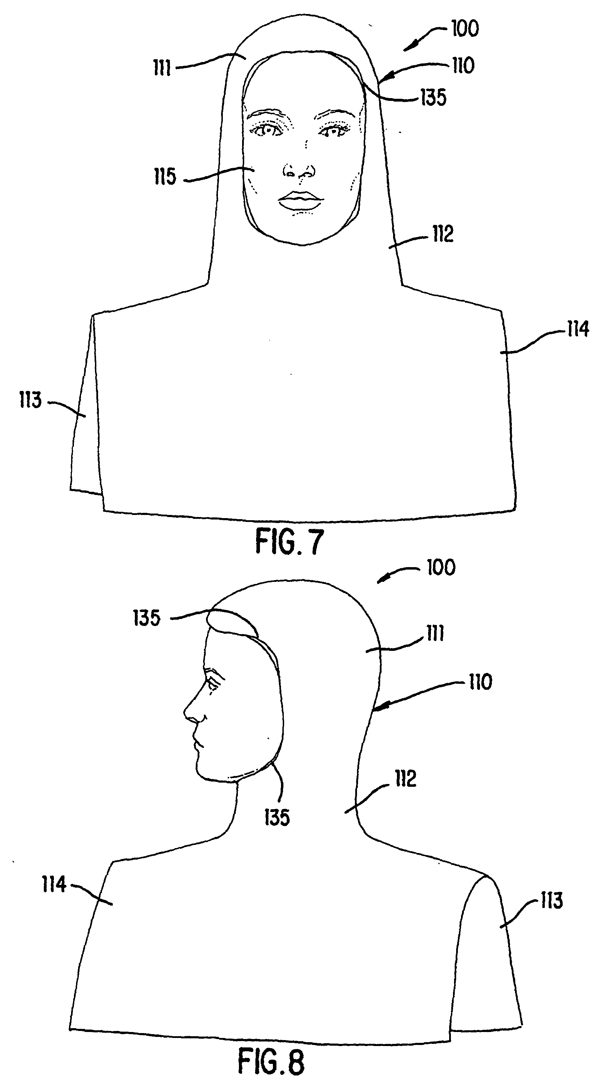

Figs. 7 and 8 disclose another embodiment of the cooling apparatus according to the invention. The cooling apparatus 100 of Figs. 7 and 8 is in the style of a poncho-shaped enveloping member 110. In addition to having a head-enveloping portion 111, a neck enveloping portion 112 and an upper back-enveloping portion 113, this embodiment has a chest-enveloping portion 114. Otherwise, this embodiment may be structured the same as the embodiment of Figs. 1-6, and will not be further discussed in detail.

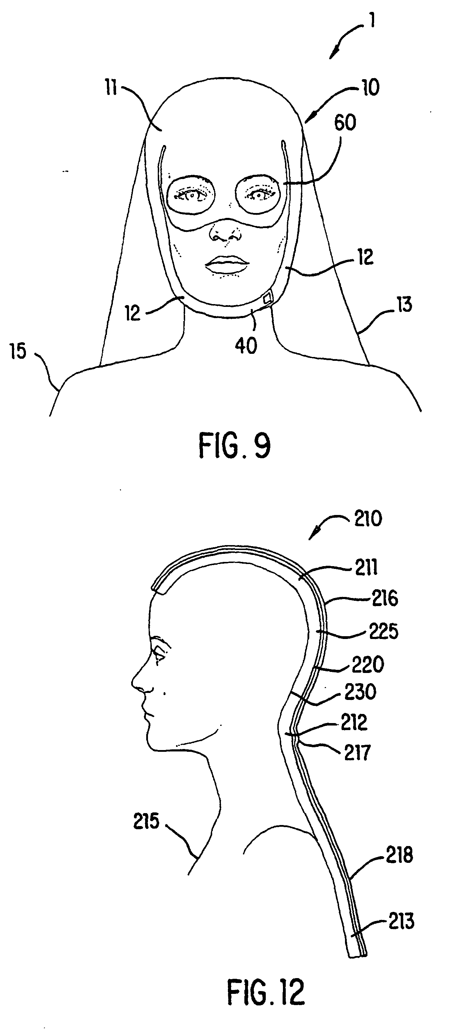

Fig. 9 shows an embodiment similar to that of Figs. 1-6 with a removable or hinged flap 60 for cooling the forehead of the patient 15. The flap may be extended down to cover the nose, cheeks, and/or chin of the patient to provide additional cooling, as long as it is provided with openings to accommodate the patient's mouth and nostrils. The flap may be transparent to allow emergency personnel to monitor any facial wounds. Any of the embodiments discussed in this application can include the flap 60 or the like.

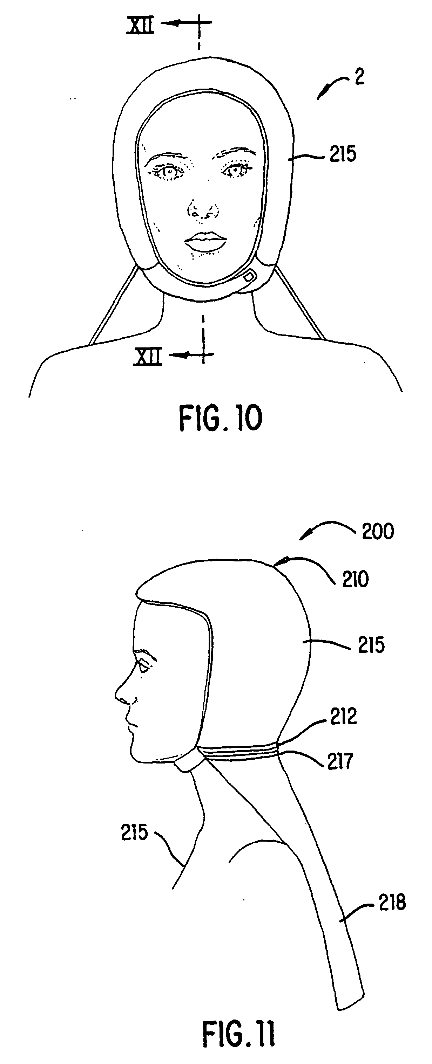

Figs. 10-12 show yet another embodiment of the invention. In this embodiment, the cooling apparatus is structured similar to that of Figs. 1-6. However, the cooling apparatus 200 is further provided with a hard outer shell in which the flexible enveloping member 210 is securely fastened by means of, for example, hooks, Velcro®, or adhesive. The hard outer shell includes a head-enveloping portion 216, a flexible neck-enveloping portion 217 and an upper back-enveloping portion 218, which correspond respectively to the head-enveloping portion 211, the neck-enveloping portion 212 and upper back-enveloping portion 213 of the flexible enveloping member 210. Where the enveloping member 210 is configured to be disposable, the hard outer shell preferably is configured to be used over and over again with different enveloping members.

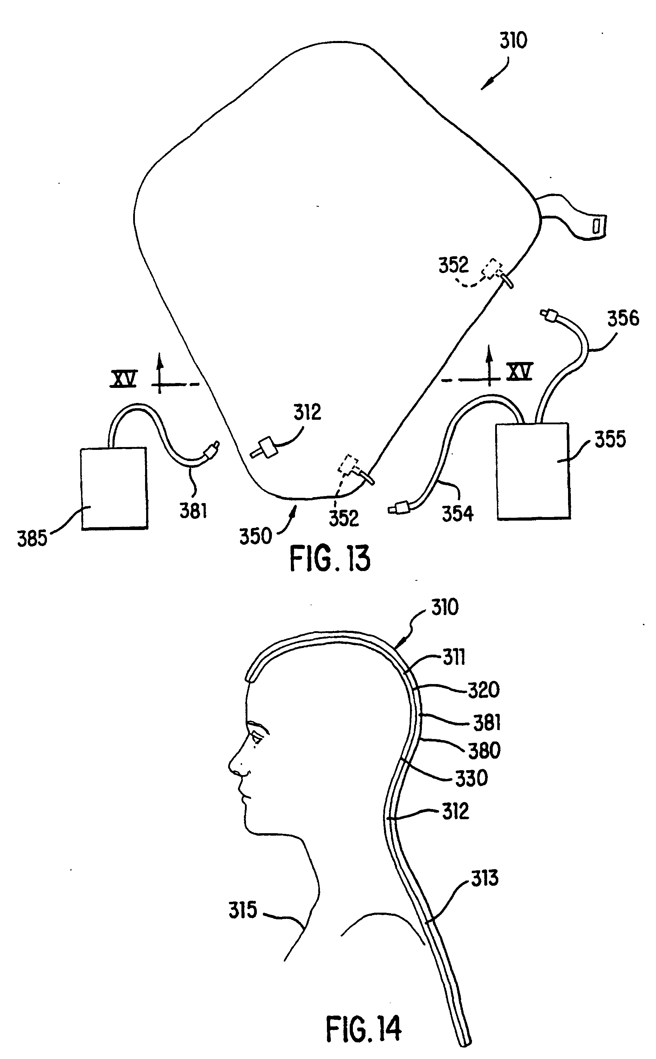

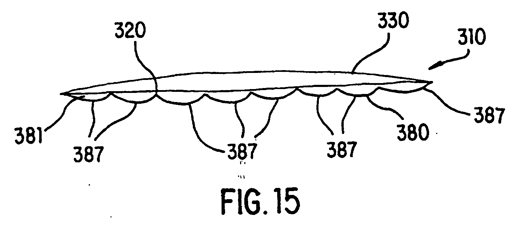

Figs. 13-15 show still another embodiment of the invention. In this embodiment, the enveloping member 310 of the cooling apparatus 300 is provided with a bladder layer 380 in addition to the outer and inner shell members 320, 330. The bladder layer 380 is sealed together to the outer and inner shell members 320, 330 to form an air bladder, or cavity 381 with the outer shell member 330. The bladder layer 380 is provided with a nipple 382 to which a gas source 385 may be attached via a tube 384 to fill the cavity 381 with a gas (i.e., air, nitrogen, helium, oxygen, or carbon dioxide) to press the outer and inner shell members 320, 330 against the head, neck and upper back of the patient 315 to provide greater contact therebetween. This contact facilitates heat transfer between the cooling apparatus 310 and the head, neck and upper back of the patient 315, and therefore provides for more rapid body cooling. Alternatively, an air bladder or bladders can be configured as a separate piece or pieces disposed on the outer shell member 330. As shown in Fig. 15, the cavity 381 can be provided with lumens 387 which cause the gas to circulate through the cavity 381 for even distribution. These lumens 387 are formed by heat sealing the bladder layer 380 to the outer shell member 330 at various places to define gas circulation paths.

In operation, the cooling apparatus of the invention sufficiently chills the brain, brain stem and associated neurologic tissues to slow their metabolism, allowing for continued resuscitation efforts. As previously stated, the invention comprises a method of treating anoxic and ischemic injuries suffered as a result of cardiac arrest, respiratory arrest, stroke or other cerebrovascular trauma, suffocation, drowning, electrocution, toxic poisoning (carbon monoxide, cyanide, etc.) metabolic insults or other similar trauma.

The operation of the cooling device according to the invention will now be discussed in detail with reference to the embodiment of Figs. 1-6; however, the remaining embodiments in this application operate similarly. Specifically, operation of the cooling apparatus 1 involves merely placing the enveloping member 10 on the patient 15 such that it envelopes the head and neck and extends down to cover the upper back of the patient 15, and securing the neck-enveloping portion 12 around the neck of the patient 15 via the closure mechanism 40. Next, the coolant source 50 is connected to the enveloping member 10 if applicable (for example, attaching the tube 54 to the nipple 52). Then, the coolant source 50 is activated. In the case of the coolant packets 53, the coolant source 50 is activated by manual manipulation of the enveloping member 10 to break the compartment separating membrane. This process is quite simple and can be performed at the trauma site by a person with minimal, if any, medical training.

Each of these embodiments of the apparatus is portable and suitable for field use, such as in ambulances, battlefields, athletic fields, aircraft, marine vehicles, spacecraft, emergency treatment facilities, and the like. They are lightweight and can be carried directly to the patient. These embodiments can also be modified for clinical (hospital type) settings. While the apparatus of the invention is preferably designed for the treatment of humans, it can also be used in treating other mammals such as dogs, horses or the like, and sized accordingly.

While the invention has been described in conjunction with specific embodiments thereof, it is evident that many alternatives, modifications and variations may be apparent to those skilled in the art. Accordingly, the preferred embodiments of the invention as set forth herein are intended to be illustrative, not limiting. Various changes may be made without departing from the spirit and scope of the invention as defined in the following claims.

## Claims

1. A portable apparatus for treating or preventing at least one of brain, brain stem and associated nervous tissue injuries in a mammal suffering from decreased blood flow to the brain, comprising:
a coolant source (62) in communication with the enveloping means, the coolant source instantaneously providing a coolant fluid chilled to a temperature sufficient to slow the metabolism of the brain, whereby when the coolant source is activated, the enveloping means becomes instantly chilled rapidly cooling the brain to a temperature sufficient to slow the metabolism of the brain a sufficient amount so that the mammal remains neurologically intact while efforts are made to restore regular blood flow to the brain of the mammal,
and **characterised by**
a flexible enveloping means for enveloping a head, neck and upper back of a mammal, the enveloping means including outer and inner shells with at least one cavity intermediate the outer and inner shells for holding a coolant fluid within the at least one cavity.

2. The apparatus according to claim 1, wherein the enveloping means is in the shape of a scarf or a poncho.

3. The apparatus according to claim 2, wherein the coolant source is disposed within the at least one cavity and comprises a packet containing chemicals which are activated upon mixing to produce the chilled coolant fluid.

4. The apparatus according to claim 1, wherein the coolant source comprises a charging mechanism disposed on an outer surface of the outer shell which upon activation produces the chilled coolant fluid.

5. The apparatus according to claim 2, wherein the coolant source comprises a portable coolant tank containing compressed liquid, the portable coolant tank being in fluid communication with the at least one cavity of the enveloping means via a tube.

6. The apparatus according to claim 5, wherein the compressed liquid is selected from the group consisting of carbon dioxide, freon and nitrogen.

7. The apparatus according to claim 2, wherein the coolant source comprises a portable coolant refrigeration unit in fluid communication with the at least one cavity of the enveloping means via inlet and outlet tubes.

8. The apparatus according to claim 2, wherein the coolant source comprises a chemical disposed within the at least one cavity which produces the chilled coolant fluid when activated by another chemical.

9. The apparatus according to claim 8, wherein the chemical comprises ammonium nitrate which produces the chilled coolant fluid when activated by water.

10. The apparatus according to claim 2, further comprising at least one temperature sensor in communication with the enveloping means that senses a temperature of the chilled coolant fluid within the enveloping means.

11. The apparatus according to claim 1, further comprising a removable or hinged flap for cooling at least a portion of the face of a mammal.

12. The apparatus according to claim 1, further comprising a hard outer shell configured to house the flexible enveloping means.

13. The apparatus according to claim 1, further comprises means for pressing the enveloping means against the head, neck and upper back of a mammal, whereby the head, neck and upper back of the mammal are rapidly cooled.

14. The apparatus according to claim 1, wherein the enveloping means further comprises:
at least one inflatable bladder; and
an inflation device in communication with the at least one inflatable bladder, wherein the at least one inflatable bladder is inflated by the inflation device to press the enveloping means against the head, neck and upper back of a mammal.

15. The apparatus according to claim 1, wherein the enveloping means is in the shape of a scarf.

16. The apparatus according to claim 1, wherein the enveloping means is in the shape of a poncho.

## Patentansprüche

1. Tragbare Vorrichtung zur Behandlung oder zur Prävention von zumindest einer der folgenden Schädigungen, nämlich des Hirns, des Hirnstamms und des assoziiertem Nervengewebes bei einem Säuger, der an verringertem Blutfluß zum Hirn leidet, mit einer Quelle an Kühlmittel (62), die mit Umhüllungsmitteln in Verbindung steht, wobei die Quelle an Kühlmittel augenblicklich eine Kühlflüssigkeit bereitstellt, die auf eine Temperatur abgekühlt ist, die ausreichend ist, um den Metabolismus des Hirns zu verlangsamen, wobei, falls die Quelle an Kühlmittel aktiviert wird, die Umhüllungsmittel augenblicklich abgekühlt werden, wodurch das Hirn rasch auf eine Temperatur abgekühlt wird, die ausreichend ist, den Metabolismus des Hirns auf einen solchen ausreichenden Grad zu verlangsamen, daß der Säuger neurologisch intakt bleibt, währenddessen Anstrengungen gemacht werden, um den regulären Blutfluß zum Hirn des Säugers wiederherzustellen, und ist **gekennzeichnet durch**
flexible Umhüllungsmittel zum Umhüllen des Kopfes, des Nackens und des oberen Rückens eines Säugers, wobei die Umhüllungsmittel einen äußeren und einen inneren Mantel einschließen, wobei zumindest ein Hohlraum zwischenliegend zwischen dem äußeren und dem inneren Mantel vorgesehen ist, um eine Kühlflüssigkeit in dem zumindest einen Hohlraum zu halten.

2. Vorrichtung nach Anspruch 1, bei der die Umhüllungsmittel in der Form eines Kopftuches oder eines Ponchos bestehen.

3. Vorrichtung nach Anspruch 2, bei der die Quelle an Kühlmittel in dem zumindest einen Hohlraum angeordnet ist und eine Packung aufweist, die Chemikalien enthält, die beim Vermischen derart aktiviert werden, daß sie die gekühlte Kühlflüssigkeit erzeugen.

4. Vorrichtung nach Anspruch 1, bei der die Quelle an Kühlmittel einen Beschickungsmechanismus aufweist, der an der Außenseite des äußeren Mantels angeordnet ist, welcher nach Aktivieren die gekühlte Kühlflüssigkeit erzeugt.

5. Vorrichtung nach Anspruch 2, bei der die Quelle an Kühlmittel einen tragbaren Kühltank aufweist, der eine unter Druck stehende Flüssigkeit aufweist, wobei der tragbare Kühltank über eine Leitung mit dem zumindest einen Hohlraum der Umhüllungsmittel strömungstechnisch in Verbindung steht.

6. Vorrichtung nach Anspruch 5, bei der die unter Druck stehende Flüssigkeit ausgewählt ist aus der Gruppe bestehend aus Kohlendioxid, Freon und Stickstoff.

7. Vorrichtung nach Anspruch 2, bei der die Quelle an Kühlmittel eine tragbare kühlende Kälteeinheit aufweist, die mit dem zumindest einen Hohlraum der Umhüllungsmittel über Einlaß- und Auslaßleitungen in strömungstechnischer Verbindung steht.

8. Vorrichtung nach Anspruch 2, bei der die Quelle an Kühlmittel eine Chemikalie aufweist, die in dem zumindest einen Hohlraum angeordnet ist, welche die abgekühlte Kühlflüssigkeit erzeugt, wenn diese durch eine andere Chemikalie aktiviert wird.

9. Vorrichtung nach Anspruch 8, bei der die Chemikalie Ammoniumnitrat aufweist, das die abgekühlte Kühlflüssigkeit erzeugt, wenn dieses mit Wasser aktiviert wird.

10. Vorrichtung nach Anspruch 2, welche ferner zumindest einen mit den Umhüllungsmitteln in Verbindung stehenden Temperatursensor aufweist, der die Temperatur der abgekühlten Kühlflüssigkeit in den Umhüllungsmitteln erfaßt.

11. Vorrichtung nach Anspruch 1, die ferner einen abnehmbaren oder angelenkten Lappen zur Kühlung von zumindest einem Bereich des Gesichtes des Säugers aufweist.

12. Vorrichtung nach Anspruch 1, die ferner einen harten äußeren Mantel aufweist, der so ausgebildet ist, daß dieser die flexiblen Umhüllungsmittel. aufnimmt.

13. Vorrichtung nach Anspruch 1, die ferner Mittel aufweist, um die Umhüllungsmittel gegen den Kopf, den Nacken oder den oberen Rücken des Säugers zu pressen, wodurch der Kopf, der Nacken und der obere Rücken des Säugers rasch abgekühlt werden.

14. Vorrichtung nach Anspruch 1, bei der die Umhüllungsmittel ferner folgendes aufweisen:
zumindest eine aufblasbare Blase; und
eine Aufblasvorrichtung, die mit der zumindest einen aufblasbaren Blase in Verbindung steht, wobei die zumindest eine aufblasbare Blase durch die Aufblasvorrichtung so aufgeblasen wird, daß die Umhüllungsmittel gegen den Kopf, den Nacken und den oberen Rücken des Säugers gepreßt werden.

15. Vorrichtung nach Anspruch 1, bei der die Umhüllungsmittel die Form eines Hals-, Kopf-, Schultertuches aufweisen.

16. Vorrichtung nach Anspruch 1, bei der die Umhüllungsmittel die Form eines Umhanges aufweisen.

## Revendications

1. Appareil portatif pour traiter ou prévenir au moins une lésion du cerveau, du tronc cérébral et/ou des tissus neurologiques associés chez un mammifère souffrant d'une diminution de l'afflux sanguin au cerveau, comprenant :
une source de réfrigérant (62) en communication avec le moyen d'enveloppe, la source de réfrigérant fournissant instantanément un fluide réfrigérant refroidi à une température suffisante pour ralentir le métabolisme du cerveau, ce par quoi, lorsque la source de réfrigérant est activée, le moyen d'enveloppe devient instantanément refroidi en refroidissant rapidement le cerveau à une température suffisante pour ralentir le métabolisme du cerveau dans une mesure suffisante pour que le mammifère reste intact du point de vue neurologique, tandis que l'on s'efforce de restaurer un flux sanguin régulier au cerveau du mammifère,
et **caractérisé par**
un moyen d'enveloppe flexible pour envelopper la tête, le cou et la partie supérieure du dos d'un mammifère, le moyen d'enveloppe comportant des coques extérieure et intérieure avec au moins une cavité entre les coques intérieure et extérieure pour retenir un fluide réfrigérant dans l'au moins une cavité.

2. Appareil selon la revendication 1, dans lequel le moyen d'enveloppe est en forme d'écharpe ou de poncho.

3. Appareil selon la revendication 2, dans lequel la source de réfrigérant est disposée dans au moins une cavité et comprend un paquet contenant des substances chimiques qui sont activées lors de leur mélange pour produire le fluide réfrigérant refroidi.

4. Appareil selon la revendication 1, dans lequel la source de réfrigérant comprend un mécanisme de charge disposé sur une surface extérieure de la coque extérieure, qui, lors de l'activation, produit le fluide réfrigérant refroidi.

5. Appareil selon la revendication 2, dans lequel la source de réfrigérant comprend un réservoir de réfrigérant portable contenant du liquide sous pression, le réservoir de réfrigérant portable étant en communication fluidique avec l'au moins une cavité du moyen d'enveloppe, par le biais d'un tube.

6. Appareil selon la revendication 5, dans lequel le liquide sous pression est choisi parmi le groupe constitué du dioxyde de carbone, du fréon et de l'azote.

7. Appareil selon la revendication 2, dans lequel la source de réfrigérant comprend une unité de réfrigération de réfrigérant portable en communication fluidique avec l'au moins une cavité du moyen d'enveloppe, par le biais de tubes d'entrée et de sortie.

8. Appareil selon la revendication 2, dans lequel la source de réfrigérant comprend une substance chimique disposée dans l'au moins une cavité qui produit le fluide réfrigérant refroidi lorsqu'elle est activée par une autre substance chimique.

9. Appareil selon la revendication 8, dans lequel la substance chimique comprend du nitrate d'ammonium qui produit le fluide réfrigérant refroidi lorsqu'il est activé par de l'eau.

10. Appareil selon la revendication 2, comprenant en outre au moins un capteur de température en communication avec le moyen d'enveloppe, et qui détecte une température du fluide réfrigérant refroidi à l'intérieur du moyen d'enveloppe.

11. Appareil selon la revendication 1, comprenant en outre un volet amovible ou articulé pour refroidir au moins une portion de la face d'un mammifère.

12. Appareil selon la revendication 1, comprenant en outre une coque extérieure dure configurée pour recevoir le moyen d'enveloppe flexible.

13. Appareil selon la revendication 1, comprenant en outre un moyen pour presser le moyen d'enveloppe contre la tête, le cou et la partie supérieure du dos d'un mammifère, ce par quoi la tête, le cou et la partie supérieure du dos du mammifère sont rapidement refroidis.

14. Appareil selon la revendication 1, dans lequel le moyen d'enveloppe comprend en outre :
au moins une vessie gonflable ; et
un dispositif de gonflage en communication avec l'au moins une vessie gonflable, l'au moins une vessie gonflable étant gonflée par le dispositif de gonflage pour presser le moyen d'enveloppe contre la tête, le cou et la partie supérieure du dos d'un mammifère.

15. Appareil selon la revendication 1, dans lequel le moyen d'enveloppe est en forme d'une écharpe.

16. Appareil selon la revendication 1, dans lequel le moyen d'enveloppe est en forme de poncho.
